# EUROPEAN PATENT APPLICATION

(11) **EP 3 418 745 A1**
(43) Date of publication of application: **26.12.2018**
(21) Application number: 17753244.7
(22) Date of filing: 15.02.2017
(51) Int. Cl.: G01N 33/574, G01N 33/68, C12Q 1/68, G01N 21/64, G01N 33/58

(54) **INFORMATION ACQUISITION METHOD FOR DIAGNOSIS OR TREATMENT OF CANCER OR IMMUNE SYSTEM-RELATED DISEASES**

(30) Priority: 19.02.2016 JP 2016030420
(71) Applicant: Konica Minolta, Inc., Tokyo 100-7015 (JP)
(72) Inventor: TAKAHASHI, Masaru, Tokyo 100-7015 (JP); FUTAYA, Etsuko, Tokyo 100-7015 (JP); OKADA, Hisatake, Tokyo 100-7015 (JP); KOYAMA, Noboru, Tokyo 100-7015 (JP)
(74) Representative: Henkel, Breuer & Partner
(86) International application number: PCT/JP2017/005589
(87) International publication number: WO 2017/141988

(57) **Abstract**

Provided is a method for acquiring information for the diagnosis or treatment of mainly cancer or immune system-related diseases, said method being characterized by including a step in which the expression level of a cancer-related protein or nucleic acid in a tumor cell is quantitated using a specimen derived from human or other tumor tissue, and/or a step in which the expression level of a protein or nucleic acid in an immunocyte is quantitated.

## Description

### Technological Field

The present invention relates to a method of obtaining information for the diagnosis or treatment, mainly of cancer or an immune system-related disease.

### Background

In recent years, immune checkpoint-related antibody drugs including an anti-PD-1 antibody, Nivolumab, have been developed, and the publishing of related articles and clinical trials are actively performed. Immune checkpoint mechanisms are mechanisms which operate via the interaction between PD-L1 expressed by cancer cells and PD-1 expressed by immune cells (activated killer T cells and the like), namely, via the PD-L1/PD-1 pathway, and they have been shown to function as immune escape mechanisms of cancer in the microenvironment around tumors. Based on this finding, anti-PD-1 antibodies and anti-PD-L1 antibodies have been developed, for the purpose of inhibiting the PD-L1/PD-1 pathway.

This is recognized by many of anticancer drug developers as an epoch-making event in the history of anticancer drug development, and immune checkpoint-related antibody drugs have grown to be one of three pillars of molecular target drugs. Recently, many companies provide expression analysis technologies and/or products in which cancer-associated genes encoding cancer-associated proteins are classified in three groups and expression analyses are carried out based on gene panels of these genes. For example, an analysis system called nCounter is now available, in which cancer-associated genes are divided in three gene panels: Pathway (pathway-related) gene panel; Immune (immune-related) gene panel; and Progression (metastasis-related) gene panel; and an expression analysis for each of the genes is provided based on these panels. The cancer-associated gene expression panels provided by nCounter: Pathway gene panel, Immune gene panel, and Progression gene panel each covers 770 genes. Proteins produced by the transcription of Immune genes, Pathway genes, and Progression genes can be defined as "immune-related proteins in cancer cells", "pathway-related proteins in cancer cells", and "metastasis-related proteins in cancer cells", respectively. There are, of course, mutant genes of these genes, and accordingly, mutant proteins corresponding to such mutant genes also exist. The mutant proteins corresponding to these mutant genes can also be included in the immune-related proteins, pathway-related proteins, and metastasis-related proteins. For example, a PDL1 mutant protein has first been reported in 2016 (Nature. 2016 Jun 16; 534 (7607): 402-6), and it is expected that useful cancer-associated proteins and mutants thereof will be increasingly reported.

Efforts are underway to provide novel immune checkpoint-related antibody drugs to patients, by utilizing the analysis results of such genes as indices or evidences for the development of anticancer drugs. Further, in order to improve the effects of pre-existing immune checkpoint-related antibody drugs, clinical trials for treatment methods are also in progress, in which a pathway-related antibody drug in another area, for example, is used in combination.

The above described nivolumab is characterized, for example, by having a high response rate and a prolonged effect, differing from conventional immunotherapies. Nivolumab has been approved by FDA, since it has been proven effective in melanoma patients with metastasis whose tumors are difficult to be surgically removed, and it is now a certified treatment. Further, it has been known, in recent years, that the above described antibody drugs are effective also in patients with non-small cell lung cancer (NSCLC), and the studies and clinical trials therefor have been actively performed.

In view of such a background, pharmaceutical companies are working on the development of companion diagnostic agents targeting PD-L1 protein, which is a ligand for PD-1 protein, so as to allow for setting the administration standard for PD-1 antibody drugs, and various types of clinical trials are underway to expand the applicability of such drugs, not only to terminally ill patients, such as those with melanoma. On the other hand, such diagnostic agents are associated with problems, such as follows, and various discussions have been made. For example, respective companies are using different types of antibodies, and focusing on different locations of expression, such as, for example, whether to evaluate PD-L1 expressed in cancer cells or PD-1 expressed in infiltrating lymphocytes. Further, in the immunostaining method (DAB method) based on a conventional enzymatic method, evaluations are carried out based on the expression rate, and there is a problem at which percentage the cut-off value should be set.

In addition, immune mechanisms are extremely complex, and it is thought that, even if the expression level of PD-L1 is evaluated, the correlation with the drug efficacy may not be obtained unless other information regarding T cells and the like is obtained.

Studies of miRNAs (microRNAs) in immune cells and cancer cells are increasingly reported, in recent years, and it is expected that obtaining the information on these nucleic acids may lead to further useful information. For example, in immune cells, miR-4717 has been reported to interact with the UTR (untranscribed region) of PD-1 to inhibit translation, and thereby inhibiting the expression of PD-1 (Oncotarget. 2015 Aug 7; 6 (22): 18933-44).

In recent years, methods for labeling proteins and/or nucleic acids have been proposed, which utilize nanosized fluorescent particles, for example, particles (Phosphor Integrated Dots: PIDs) obtained by integrating phosphors such as fluorescent dyes or quantum dots, using a resin, etc., as a matrix, and efforts are being made for realizing the practical use thereof. By labeling a target protein and/or a target nucleic acid using phosphor integrated dots, and irradiating an excitation light corresponding to the fluorescent substance, the phosphor integrated dots indicate the number and the positions of the molecules of the protein and/or the nucleic acid with a high accuracy, and enable these molecules to be observed as bright spots with high brightness. Further, observation and imaging can be performed for a relatively long period of time, since the fluorescence is less prone to discoloration. For example, WO 2012/029752 (Patent Document 1), WO 2013/035703 (Patent Document 2) and the like disclose methods in which immunostaining of target proteins is carried out using phosphor integrated dots.

### Related Art Documents

### Patent Documents

Patent Document 1: WO 2012/029752
Patent Document 2: WO 2013/035703

### Non-patent Document

Non-patent Document 1: N Engl J Med. 2012 June 28; 366 (26): 2443-2454

### Summary

### Problems to be Solved by the Invention

An object of the present invention is to provide a method of obtaining useful information which can be used for the diagnosis or treatment, mainly of cancer or an immune system-related disease. Means for Solving the Problems

The present inventors have discovered that it is possible to obtain useful information as descried above: by quantifying the expression level of a cancer-associated protein in a tumor cell, using a specimen derived from tumor (cancer) tissue of a human or a non-human, and/or the expression level of a protein in an immune cell of a human or a non-human, preferably, by immunostaining using fluorescent nanoparticles such as phosphor integrated dots; and by utilizing information obtained, for example, from the average expression level per cell of a target protein, a histogram showing the expression level per cell of the protein and the number of cells (frequency) corresponding thereto, and the like. Further, the present inventors have also discovered that useful information can further be obtained: by measuring the distance between the tumor cell and the immune cell, or the distance between immune cells which interact with each other; or by quantifying a nucleic acid, a cytokine or a membrane vesicle-associated protein, present in the specimen derived from tumor tissue of a human or a non-human, or in a specimen of extracellular vesicles, lymph node, blood or body fluid, of a human or a non-human.

In other words, the present invention provides, in one aspect, a method of obtaining information for diagnosis or treatment, the method including the step of quantifying the expression level of a cancer-associated protein in a tumor cell, using a specimen derived from tumor tissue of a human or a non-human, and preferably, further including the step of quantifying the expression level of a miRNA contained in an immune cell, for example, which miRNA regulates the expression level of the cancer-associated protein. The present invention provides, in another aspect, a method of obtaining information for diagnosis or treatment, the method including the step of quantifying the expression level of a protein in an immune cell, using a specimen derived from tumor tissue of a human or a non-human, and preferably, further including the step of quantifying the expression level of a miRNA contained in a tumor cell, for example, which miRNA regulates the expression level of the protein.

### Effect of the Invention

Since the present invention allows for quantifying the expression level of a specific type of protein in a specific type of cell, preferably, further quantifying the expression level of a specific type of a miRNA, with a high accuracy, using phosphor integrated dots or the like, it becomes possible to obtain useful information for the diagnosis or treatment of cancer or an immune system-related disease, which could not be found out by a technique, such as DAB, in which the evaluation is carried out based on the expression rate (the ratio of the cells in which the expression of a specific protein is observed, among the cells of a specific type). In addition, by combining pieces of information (factors) other than the information described above, a more detailed stratification of patients can be performed, thereby enabling to provide various administration standards.

### Detailed Description of Embodiments

The method of obtaining information for diagnosis or treatment of cancer or an immune system-related disease, according to the present invention (in the present specification, sometimes simply referred to as the "method of obtaining information according to the present invention") includes at least one, preferably both, of the step of quantifying the expression level of a cancer-associated protein in a tumor cell, using a specimen derived from tumor tissue of a human or a non-human, and the step of quantifying the expression level of a protein in an immune cell, using the specimen. The cancer-associated protein in a tumor cell and the protein in an immune cell are each sometimes referred to as a "target protein" in the present specification.

In a preferred embodiment of the method of obtaining information according to the present invention, the method further includes at least one, preferably both, of the step of quantifying, using the specimen derived from tumor tissue of a human or a non-human, a miRNA contained in an immune cell or a miRNA contained in a tumor cell, which miRNA regulates the expression level of the target protein (the cancer-associated protein in the tumor cell or the protein in the immune cell). The miRNA contained in the immune cell or the miRNA contained in the tumor cell, or another nucleic acid selected depending on the objective, is sometimes referred to as a "target nucleic acid" in the present specification.

The type of the "cancer or an immune system-related disease" and the content of "information for diagnosis or treatment" are not particularly limited. The present invention can be used for any cancer or immune system-related disease, as long as the quantification of a protein or a nucleic acid expressed in a cancer (tumor) cell(s) or an immune cell(s) allows for obtaining information regarding diagnosis, such as, for example, whether or not the donor of the cells is affected by the disease and how far the disease has progressed (cancer stage, and the like), or information regarding treatment, such as the extent of the efficacy of a certain drug (particularly, a molecular target drug) for the disease. In the present invention, in cases where the expression level of a cancer-associated protein in a tumor cell(s) is quantified, for example, an embodiment is used which is intended mainly for a tumor; whereas in cases where the expression level of a protein in an immune cell(s) is quantified, an embodiment is used which is intended not only for a tumor but also for another immune system-related disease.

The "tumor tissue" may be derived from a tumor of a human (cancer patient), or may be derived from a tumor of an animal other than a human.

The "specimen derived from tumor tissue" refers to a lesion site, such as a specimen collected from tumor tissue, or cells obtained by culturing the tumor cells contained in the collected specimen, and is usually in the form of a sample slide prepared in accordance with a predetermined procedure, as commonly used in the case of evaluating the expression level of a target protein by immunostaining, or the like.

It is also possible to use a specimen derived from tumor tissue of an experimental animal, as the "specimen derived from tumor tissue". The "experimental animal" as used in the present specification is one generally referred to as a tumor-bearing animal. For example, tumor-bearing mice can be largely categorized into three types: naturally induced tumor-bearing mice, cultured cancer cell-transplanted mice, and patient-derived tumor-transplanted mice (see the following table; Kohrt et al., Defining the optimal murine models to investigate immune checkpoint blockers and their combination with other immunotherapies. Annals of Oncology 00: 1-9, 2016).

**[Table A]**

| | Cancer cells | Immune cells | Model |
|---|---|---|---|
| Naturally induced tumor-bearing mice | murine | murine | (0) Classic model produced by transplanting a carcinogen compound |
| | | | (1) *Genetic-engineered mouse model |
| | | | (2) *Human KI mice |
| Cultured cancer cell-transplanted mice | murine | murine | (3) Syngeneic murine model |
| | human | murine | (4) Cell-line derived xenograft (CDX) |
| Patient-derived tumor tissue-transplanted mice | human | murine | (5) Patient derived xenograft (PDX) |
| | | | (6) Immuno-avatar mice |
| | | | (7) Hemato-lymphoid humanized mice |
| | | | (8) Immune-PDX |

| | | | |
|---|---|---|---|
| *gene knock-in mice | | | |

The definition of the "experimental animal" encompasses: an experimental animal of the 0th generation transplanted with tumor (cancer) tissue or tumor (cancer) cells collected from a human (cancer patient), or transplanted with human derived tumor cells which are established as a cultured cell line; and an experimental animal of the nth generation (n ≥ 1) transplanted with the tumor tissue or tumor cells, originated from the tumor tissue or tumor cells which had been transplanted into the 0th generation as described above, and grown within the body of an experimental animal of the n-1 generation. Such an experimental animal can be produced by a known technique.

Examples of naturally induced tumor-bearing mice include: mice of a classic model obtained by transplanting a carcinogenic compound, mice of a genetic-engineered mouse model, and Human KI mice (both of the latter two are gene knock-in mice). Examples of cultured cancer cell-transplanted mice include mice of a syngeneic murine model and CDX (Cell-line derived xenograft) model mice. Examples of patient-derived tumor-transplanted model mice include: PDX (Patient derived xenograft) model mice, Immuno-avatar model mice, Hemato-lymphoid humanized model mice, and Immune-PDX model mice. Various types of tumor-bearing model mice as described above can be produced, and already-produced tumor-bearing mice are also commercially available. On the other hand, tumor-bearing model mice transplanted with cultured cells derived from tumor cells collected from patients are of a more classical model, and can be produced easily.

### (Cancer-associated Proteins in Tumor Cells)

Representative examples of the "cancer-associated protein" include "immune-related proteins in cancer cells", "pathway-related proteins in cancer cells", and "metastasis-related proteins in cancer cells". Various types of cancer-associated proteins are known for each of the proteins categorized as described above. The cancer-associated protein can be selected as appropriate depending on the purpose of the diagnosis or treatment, without particular limitation. Cancer-related gene expression panels provided by nCounter: an immune-related gene panel (Immune), a pathway-related gene panel (Pathway), and a metastasis-related gene panel (Progression), each covers 770 genes, and the proteins coded by the genes of these three panels correspond to the immune-related proteins, the pathway-related proteins, and the metastasis-related proteins in cancer cells, respectively.

Examples of the "immune-related proteins in cancer cells" include immune checkpoint proteins, such as TL1A, GITR-L, 4-188-L, CX4D-L, CD70, HHLA2, ICOS-L, CD85, MHC-II, PDL2, BTNL2, B7-H4, CD48, HVEM, CD40L, TNFRSF25, GITR, 4-188, OX40, CD27, TMIGD2, ICOS, CD28, TCR, LAG3, CTLA4, PD1, CD244, TIM3, BTLA, CD160, LIGHT, PD-L1, CD40, CD80, CD86, VISTA, and B7-H3.

Examples of the "pathway-related proteins in cancer cells" include: cancer cell growth factors and cancer cell growth factor receptors, such as EGFR (HER1), HER2, HER3, HER4, IGFR, and HGFR; cell surface antigens, vascular growth factors and vascular growth factor receptors, such as VEGF-A, VEGF-B, VEGF-C, VEGF-D, VEGF-E, PlGF-1, and PlGF-2; and interferons, interleukins, G-CSF, M-CSF, EPO, SCF, EGF, FGF, IGF, NGF, PDGF, and TGF.

Examples of the "metastasis-related proteins in cancer cells" include: ACTG2, ALDOA, APC, BRMS1, CADM1, CAMK2A, CAMK2B, CAMK2D, CCL5, CD82, CDKN1A, CDKN2A, CHD4, CNN1, CST7, CTSL, CXCR2, YBB, DCC, DENR, DLC1, EGLN2, EGLN3, EIF4E2, EIF4EBP1, ENO1, ENO2, ENO3, ETV4, FGFR4, GSN, HK2, HK3, HKDC1, HLA-DPB1, HUNKIL11, KDM1A, KISS1, LDHA, L1FR, MED23, MET, MGAT5, MAP2K4, MT3, MTA1, MTBP, MTOR, MYCL, MYH11, NDRG1, NF2, NFKB1, NME1, NME4, NOS2, NR4A3, PDK1, PEBP4, PFKFB1, PFKFB4, PGK1, PLAUR, PTTG1, RB1, RORB, SET, SLC2A1, SNRPF, SSTR2, TCEB1, TCEB2, TCF20, TF, TLR4, TNFSF10, TP53, TSHR, MMP, MMP2, MMP10, and HIF1.

### (Proteins in Immune Cells)

Examples of the "protein in an immune cell" include PD-1, CTLA-4, TIM3, Foxp3, CD3, CD4, CD8, CD25, CD27, CD28, CD70, CD40, CD40L, CD80, CD86, CD160, CD57, CD226, CD112, CD155, OX40 (CD134), OX40L (CD252), ICOS (CD278), ICOSL (CD275), 4-1BB (CD137), 4-1BBL (CD137L), 2B4 (CD244), GITR (CD357), B7-H3 (CD276), LAG-3 (CD223), BTLA (CD272), HVEM (CD270), GITRL, Galectin-9, B7-H4, B7-H5, PD-L2, KLRG-1, E-Cadherin, N-Cadherin, R-Cadherin, IDO, TDO, CSF-1R, HDAC, CXCR4, FLT-3, and TIGIT.

Examples of information which can be obtained by the quantification of the expression level of a cancer-associated protein or a nucleic acid in a tumor cell(s), and/or the quantification of the expression level of a protein or a nucleic acid in an immune cell(s) include information regarding: (i) the average expression level per cell of the target protein, and preferably, that of the target nucleic acid; (ii) the expression level per unit area of the tissue, of the target protein, and preferably, that of the target nucleic acid; (iii) a histogram showing the expression level per cell of the target protein, and preferably that of the target nucleic acid, in addition, and the number of cells corresponding thereto; (iv) a curve showing the expression level per cell of the target protein, and preferably, that of the target nucleic acid in addition, and the number of cells corresponding thereto;in a specimen (sample slide) derived from tumor tissue of a human or a non-human. One of these pieces of information may be used alone, or a plurality of pieces of information may be used in combination.

In the case of quantifying (i) the average expression level per cell of the target protein, for example, a specimen (sample slide) is immunostained with fluorescent nanoparticles, and at the same time, also stained with a staining agent for morphological observation (such as eosin) so that the shape of the cells can be identified. The observation and imaging of the specimen in a dark field are carried out while irradiating an excitation light having a predetermined wavelength corresponding to the fluorescent nanoparticles, to obtain an image in which the fluorescent nanoparticles labeling the target protein are shown as bright spots. Meanwhile, the observation and imaging in a bright field are carried out, to obtain an image in which the shape of the cells are indicated by the staining. When the thus obtained two images are overlaid by image processing, it is possible to count the number of bright spots indicating the molecules of the expressed target protein, for each of the cells included in the entire image, or included in a specific area (for example, tumor tissue alone) in the image. The number of bright spots may be used as an index of the expression level of the target protein. Further, there is a case in which a plurality of fluorescent nanoparticles constitute one single bright spot, and in such a case, the number of fluorescent nanoparticles included in the one single bright spot can be calculated by dividing the brightness (brightness, fluorescence intensity) thereof by the brightness per one fluorescent nanoparticle separately measured in advance. The thus obtained number of the particles may be used as an index value of the expression level of the target protein.
By determining the number of bright spots or the number of particles for all the cells included in the image, it is possible to quantify the average expression level per cell.

By carrying out nucleic acid staining, instead of the immunostaining, in (i), it is possible to quantify the average expression level per cell of the target nucleic acid.

In the case of determining (ii) the expression level per unit area of the tissue, of the target protein or the target nucleic acid, it can be achieved by: obtaining the total sum of the number of bright spots or the number of particles determined in the same manner as in (i), in the cells contained in the tissue present in a specific area in the image; and then dividing the sum by the area of the tissue.

In the case of preparing a (iii) a histogram showing the expression level per cell of the target protein or the target nucleic acid and the number of cells corresponding thereto, first, the number of bright spots or the number of particles indicating the molecules of the expressed target protein or target nucleic acid is obtained, for each of the cells included in the entire image, or included in a specific area (for example, tumor tissue alone) of the image, in the same manner as in (i). Subsequently, the expression level per cell of the target protein is divided into sections every predetermined number of particles (for example, as carried out in the Examples in the present specification, the number of particles per cell of from one to 300 is divided every 20 particles, into 16 sections, including the section of 0) and plotted on the horizontal axis, and the number of cells (frequency) corresponding to each section is counted and plotted on the vertical axis, thereby preparing the histogram.

In the case of preparing (iv) a curve showing the expression level per cell of the target protein or the target nucleic acid and the number of cells corresponding thereto, first, the number of bright spots or the number of particles indicating the molecules of the expressed target protein or target nucleic acid is obtained, for each of the cells included in the entire image, or included in a specific area (for example, tumor tissue alone) of the image, in the same manner as in (i). Subsequently, the expression level per cell of the target protein or the target nucleic acid is plotted on the horizontal axis, continuously (without dividing into sections as in the case of preparing the histogram), and the number of cells (frequency) corresponding to each expression level is counted and plotted on the vertical axis, thereby preparing the curve.

From the histogram described in (iii) and the curve described in (iv), it is possible to obtain information regarding, for example, the state of the distribution (the shape of the histogram or the curve, the number of the peaks); the levels of values of the mean value or median value and the variance (CV); and in the case of the histogram, in particular, the level of the number of cells (frequency) corresponding to the section with the highest number of bright spots or particles per cell, and the like. By comparing such information with the test results of drug efficacy, stability or the like, it is possible to analyze and to understand, for example, to which piece of information the drug efficacy, the stability, or the like is most highly related, in other words, which piece of information is most adequate to be used for making a prediction of the drug efficacy, stability, or the like.

The term "quantifying" refers to identifying the expression level and the like of the target protein or nucleic acid, using a "quantitative" technique, not a "qualitative" technique.

The "qualitative" technique refers to a technique in which the expression level of a protein or a nucleic acid and, the number of cells expressing the same, and the like, or index values closely related thereto, are not directly used, although correlated therewith; but instead, the numbers or the index values within a predetermined range are summarized and represented as one score, and about several, for example, from 2 to 5 levels of such scores are used for evaluation, based typically on the subjective and empirical judgement of the observer. For example, the IHC method using DAB staining and intended for detecting HER2 protein expressed on the cell membrane of breast cancer cells and the like, which method carries out the evaluation based on the stainability of the cell membrane of the cancer cells and the staining intensity (staining pattern) thereof according to the following four stage scores ("Guidelines for HER2 Testing, Third Edition" by Trastuzumab Pathology Committee, September, 2009), corresponds to the "qualitative" technique: 3+ (in cases where the ratio of cancer cells with an intense and complete positive staining of the cell membrane is > 30%: positive); 2+ (in cases where the ratio of cancer cells with a weak to moderate degree of complete positive staining of the cell membrane is ≥10%, or the ratio of cancer cells with an intense and complete positive staining of the cell membrane is ≥ 10% and ≤ 30%: equivocal); 1+ (in cases where the ratio of cancer cells with a barely recognizable, faint staining of the cell membrane is ≥ 10%, and the cancer cells are partially stained only at the cell membrane: negative); and 0 (in cases where no positive stain is observed in the cell membrane, or the ratio of cancer cells with a positive staining of the cell membrane is > 10% (positive staining localized only to the cell membrane is excluded from the evaluation): negative). Further, the technique disclosed in Non-patent Document 1 (page 20527, Figure 3) in which the expression level of a protein is evaluated according to four stage scores, based on a stained image obtained by the IHC method, also corresponds to the "qualitative" technique.

On the other hand, the "quantitative" technique refers to a technique in which the expression level of a protein or a nucleic acid and the number of cells expressing the same, or index values closely related thereto, are directly used, and typically refers to a method based on objective measured results obtained using an apparatus. Representatively, a technique is used in which a target protein or a target nucleic acid is labeled and quantified, using fluorescent nanoparticles, namely, particles having a nanosized diameter, for example, quantum dots (those which are not integrated), or particles obtained by integrating phosphors such as fluorescent dyes or quantum dots, using a resin, etc., as a matrix (Phosphor Integrated Dots: PIDs). In particular, a quantification method carried out using phosphor integrated dots (sometimes referred to as "PID method" in the present specification), which will be described later in the present specification and which is used also in the Examples, is particularly suitable as the "quantitative" technique to be used in the present invention. However, the "quantitative" technique which can be used in the present invention is not limited to the technique using fluorescent nanoparticles, such as the PID method, and other techniques having the same level of accuracy as that may also be used.

Basic embodiments of the PID method are known from the disclosures of WO 2012/029752 (Patent Document 1) and WO 2013/035703 (Patent Document 2), or other patent documents or non-patent documents. The PID method can be carried out also in the present invention, in an embodiment in accordance with the case of performing a pathology diagnosis using a sample slide, for example.

The "histogram" is prepared by dividing the expression level per cell of the target protein or the target nucleic acid into sections every predetermined number of particles, and plotting the number of cells (frequency) corresponding to each section. However, since the histogram is originally a graph obtained by measuring the expression level (the number of bright spots or the number of particles) of the protein or the nucleic acid and the number of cells expressing the protein or the nucleic acid, and directly using these values, the histogram is categorized as information obtained by a "quantitative" technique, not a "qualitative" technique.

In an example of a preferred embodiment, the method of obtaining information according to the present invention includes, in addition to the two steps as described above, one or more selected from the group consisting of: (a) the step of measuring the distance between the tumor cell and the immune cell, in the specimen derived from tumor tissue of a human or a non-human; (a') the step of measuring the distance between immune cells which interact with each other, in the specimen derived from tumor tissue of a human or a non-human; (b) the step of quantifying a nucleic acid, such as a DNA- or RNA-related substance (mRNA, tRNA, rRNA, miRNA, non-cording RNA, or the like), a cytokine, or a membrane vesicle-associated protein, present in the specimen derived from tumor tissue of a human or a non-human or in a specimen of extracellular vesicles, lymph node, blood, or body fluid (such as saliva) of a human; and (c) the step of measuring an immune score and/or carrying out the microsatellite instability test. Carrying out any of these steps using phosphor integrated dots (PIDs) is also an example of a preferred embodiment of the present invention.

By measuring the distance between the tumor cell and the immune cell, as in the step (a), it is possible to evaluate the actual degree of interaction occurring between the tumor cell and the immune cell. For example, the distance between fluorescent labels (such as bright spots of PIDs) bound to the molecules of a protein (PD-L1 or the like) specifically expressed in a tumor cell, and fluorescent labels bound to the molecules of a protein (CD8 or the like) specifically expressed in an immune cell, which distance can be measured by the image processing as will be described later, can be considered as the distance between the tumor cell and the immune cell. In the case of carrying out this step, an immunostaining treatment for the fluorescent labeling of a protein specifically expressed in tumor cells and an immunostaining treatment for the fluorescent labeling of a protein specifically expressed in immune cells (double immunostaining) may performed on the same single specimen (tissue section or the like). It is appropriate to use fluorescent labels which emit fluorescence of different wavelengths, for the respective treatments, so that the fluorescent labels can be distinguished from one another.

When measuring the distance between the tumor cell and the immune cell, as in the step (a), it is also possible to use bright spots of fluorescent labels bound to the molecules of a nucleic acid(s) specifically expressed in tumor cells and/or immune cells, instead of those bound to the molecules of a protein(s) specifically expressed in tumor cells and/or immune cells.

By using a protein and/or a nucleic acid specifically expressed in immune cells which interact with each other, for example, in each of two types of immune cells such as T cells and dendritic cells, in the step (a), instead of the protein(s) specifically expressed in tumor cells and/or immune cells, it is possible to measure the distance between these immune cells, as in the step (a'), and to evaluate the actual degree of interaction occurring between the respective types of cells.

Examples of the nucleic acid in the specimen, to be used in the step (b), include: RNA-related substances, for example, miRNAs such as miR21, miR34a, miR197, miR200, miR513, miR-133a, miR-143, exosomal micro-RNAs (miR-181c, miR-27b), let-7a, and miR-122; cytokines such as IL-1, IL-2, IL-4, IL-6, IL-10, IL-12, IL-18, IFN-α, IFN-β, IFN-γ, TNF, and TGF-β; and membrane vesicle-associated proteins such as, HSP, GAPDH, keratin, tubulin, actin, vimentin, fibrin, fibronectin, annexin, flotillin, galectin, and α-enolase.

The nucleic acid (gene) or the protein (cytokine, membrane vesicle-associated protein) to be quantified in the step (b), may be one which is present within the tumor tissue or in the vicinity of the tumor cells, namely, one present in the specimen (such as a sample slide on which a tissue section is placed) derived from tumor tissue of a human or a non-human for quantifying the expression level of a cancer-associated protein in a tumor cell and/or the expression level of a protein in an immune cell, along with the tumor cells and/or immune cells; or alternatively, one which is present in a specimen of extracellular vesicles, lymph node, blood, body fluid or the like, separately from the tumor cells and/or immune cells.

An example of a preferred embodiment of the step (b) in the present invention is an embodiment in which a miRNA contained in an immune cell or a miRNA contained in a tumor cell, which is present in the specimen derived from tumor tissue of a human or a non-human and which regulates the expression level of the cancer-associated protein in the tumor cell or the protein in the immune cell, is taken as the target nucleic acid. In recent years, miRNAs are beginning to be known to be involved in the regulation of the expression levels of various types of proteins. For example, there are cases where a specific type of miRNA (such as miR4717) contained in tumor cells binds to the untranscribed region (UTR) of the mRNA of a specific type of protein expressed in immune cells which interact with the tumor cells, and inhibits the expression of the protein. Conversely, there are cases where a miRNA contained in immune cells inhibits the expression of a cancer-associated protein expressed in tumor cells which interact with the immune cells. In cases where such a specific miRNA is contained in a large amount in a certain cell, or in cases where the distance between a cell containing a specific miRNA and a cell which interacts therewith is short, it is possible to assume that the expression level of a specific protein corresponding to the miRNA is more likely to be inhibited. Accordingly, information useful to a certain extent can be obtained by carrying out the quantification of a specific miRNA contained in an immune cell(s) or a specific miRNA contained in a tumor cell(s), alone. However, by performing the quantification of the expression level of a specific cancer-associated protein in a tumor cell(s) or a specific protein contained in an immune cell(s), in combination, it becomes possible to obtain more useful information for diagnosis or treatment.

In cases where the protein in the step (b) is present on a sample slide, the protein can be quantified by the same immunostaining as that used for quantifying a cancer-associated protein in a tumor cell and/or a protein in an immune cell, by using an appropriate primary antibody or the like; and in cases where the protein is present in a liquid specimen such as blood, the protein can be quantified by a known technique suitable for such a case. On the other hand, in cases where the nucleic acid in the step (b) is present on a sample slide, the nucleic acid can be quantified, using an appropriate probe or the like, by a technique in accordance with the FISH method; and in cases where the nucleic acid is present in a liquid specimen such as blood, the nucleic acid can be quantified by a known technique suitable for such a case. Further, in cases where these protein and nucleic acid are fluorescently labeled and quantified, fluorescent nanoparticles can be used in the same manner as in the case of quantifying the expression level of a cancer-associated protein or a nucleic acid in a tumor cell and/or that of a protein or a nucleic acid in an immune cell. In particular, phosphor integrated dots (PIDs) are preferably used.

The immune score in the step (c) is obtained based on a combination of the measured values of various types of immune parameters, and used for determining immune competence against various types of diseases. In general, the immune score is a score indicating the immune competence of an individual, obtained by a combination of the measured values. Examples of the immune parameter include: the number of T cells, the ratio of the numbers of CD4+/ CD8+ T cells, the number of naive T cells, the ratio of the numbers of naive/ memory T cells, the ratio of the numbers of CD8+/ CD28+ T cells, the number of B cells, the number of NK cells, and T cell growth coefficient. The microsatellite instability test (MSI test) is a test carried out as an auxiliary diagnosis for "Lynch syndrome (Hereditary Non-Polyposis Colorectal Cancer: HNPCC)", which is one type of hereditary colorectal cancer, and it can be claimed on medical insurance as a "malignant tumor genetic testing", since the medical fee revision in fiscal year 2006. The microsatellite instability test is a test which measures, by PCR or the like, the frequency of the phenomenon in which microsatellite repetitive sequences in tumor tissue exhibit a number of repetitions different from that in non-tumor (normal) tissue, due to a decrease in the function to repair errors in the base sequence which occurs during the replication of DNA. The test for obtaining an immune score and the microsatellite instability test are standard test, and can be carried out in accordance with known techniques. However, these tests can also be carried out by the quantification method of measuring bright spots, using fluorescent nanoparticles, preferably phosphor integrated dots (PIDs).

A more detailed description will be given below regarding techniques for quantifying a target biological substance in a specimen, representatively, regarding immunostaining using fluorescent nanoparticles.

### < Antibodies >

As the primary antibody, it is possible to use an antibody (IgG) which specifically recognizes and binds to a protein, which is the target biological substance as described above, as an antigen. For example, an anti-PD-L1 antibody can be used in cases where PD-L1 (expressed protein) is taken as the target biological substance, and an anti-CD8 antibody can be used in cases where CD8 is taken as the target biological substance.

As the secondary antibody, it is possible to use an antibody (IgG) which specifically recognizes and binds to the primary antibody as an antigen.

Each of the primary antibody and the secondary antibody may be a polyclonal antibody, but preferably, a monoclonal antibody, in order to stably carrying out the quantification. The species of an animal (immune animal) to be used for producing the antibodies is not particularly limited, and can be selected from a mouse, a rat, a guinea pig, a rabbit, a goat, sheep, and the like, as has been conventionally done.

The primary antibody does not have to be a naturally-occurring, full length antibody, and may be an antibody fragment or a derivative, as long as it is capable of specifically recognizing and binding to a specific biological substance (antigen). In other words, the term "antibody" as used in the present specification encompasses not only full length antibodies, but also antibody fragments such as Fab, F(ab)'2, Fv, and scFv, and derivatives such as chimeric antibodies (humanized antibodies and the like), and multifunctional antibodies.

### < Fluorescent Nanoparticles >

The fluorescent nanoparticles to be used in the present invention is preferably "phosphor integrated dots" (PIDs) capable of emitting fluorescence with an intensity sufficient for allowing single molecules of the target biological substance to be observed as individual bright spots.

Further, the term "phosphor" as used in the present specification refers to a substance which absorbs the energy of an electromagnetic wave (an X ray, UV light or a visible ray) with a predetermined wavelength irradiated thereto, and emits the surplus energy generated when electrons excited by the absorbed energy return to the ground state, as an electromagnetic wave, namely a substance which emits "fluorescence", and which is capable of binding directly or indirectly to the secondary antibody. The term "fluorescence" has a broad meaning, and encompasses: phosphorescence with a long luminescence lifetime, whose luminescence is sustained even when the irradiation of an electromagnetic wave for eliciting excitation is terminated; and fluorescence in the narrow sense, with a short luminescence lifetime.

### < Phosphor Integrated Dots >

The phosphor integrated dots in the present invention are nanosized particles having a structure in which a plurality of phosphors (such as fluorescent dye molecules) are encapsulated in particles composed of an organic substance or an inorganic substance, as a matrix, and/or adsorbed on the surface of the particles. In this case, it is preferred that the matrix (such as a resin) and the fluorescent substance have substituents or sites having charges opposite to each other, so that an electrostatic interaction takes place therebetween.

Among substances which can be used as the matrix as a component of the phosphor integrated dots, examples of the organic substance, include: resins generally categorized as thermosetting resins, such as melamine resins, urea resins, aniline resins, guanamine resins, phenol resins, xylene resins, and furan resins; resins generally categorized as thermoplastic resins, such as styrene resins, acrylic resins, acrylonitrile resins, AS resins (acrylonitrile-styrene copolymers), and ASA resins (acrylonitrile-styrene-methyl acrylate copolymers); other resins such as polylactic acids; and polysaccharides. Examples of the inorganic substance include silica and glass.

### • Semiconductor Integrated Nanoparticles

The semiconductor integrated nanoparticles have a structure in which semiconductor nanoparticles as phosphors are encapsulated in the above described matrix and/or adsorbed on the surface thereof. The material for constituting the semiconductor nanoparticles is not particularly limited, and examples thereof include: Group II-VI compounds, Group III-V compounds, and compounds containing Group IV elements, such as CdSe, CdS, CdTe, ZnSe, ZnS, ZnTe, InP, InN, InAs, InGaP, GaP, GaAs, Si, and Ge. In cases where the semiconductor is encapsulated in the matrix, the semiconductor may or may not be chemically bound to the matrix, as long as it is dispersed inside the matrix.

### Fluorescent Dye-Integrated Nanoparticles

The fluorescent dye-integrated nanoparticles have a structure in which a fluorescent dye is encapsulated in the above described matrix and/or adsorbed on the surface thereof. The fluorescent dye is not particularly limited, and examples thereof include rhodamine-based dye molecules, squarylium-based dye molecules, cyanine-based dye molecules, aromatic ring-based dye molecules, oxazine-based dye molecules, carbopyronine-based dye molecules, and pyrromethene-based dye molecules. Alternatively, it is possible to use Alexa Fluor (registered trademark, manufactured by Invitrogen Corporation)-based dye molecules, BODIPY (registered trademark, manufactured by Invitrogen Corporation)-based dye molecules, Cy (registered trademark, manufactured by GE Healthcare Inc.)-based dye molecules, DY-based dye molecules (registered trademark, manufactured by Dyomics GmbH), HiLyte (registered trademark, manufactured by AnaSpec, Inc.)-based dye molecules, DyLight (registered trademark, manufactured by Thermo Fisher Scientific Inc.)-based dye molecules, ATTO (registered trademark, manufactured by ATTO-TEC GmbH)-based dye molecules, MFP (registered trademark, manufactured by Mobitec GmbH)-based dye molecules, or the like. The generic names of such dye molecules are based on the primary structures (skeletons) of the compounds or the registered trademarks of the compounds, and those skilled in the art can adequately understand the ranges of the fluorescent dyes belonging to the respective types of dye molecules, without undue trials and errors. In cases where the fluorescent dye is encapsulated in the matrix, the fluorescent dye may or may not be chemically bound to the matrix, as long as it is dispersed inside the matrix. < Phosphor Integrated Dots >

The phosphor integrated dots (sometimes also referred to as "fluorescent substance-integrated nanoparticles" in other literature) can be produced in accordance with a known method (see, for example, JP 2013-57937 A).

More specifically, fluorescent substance-encapsulating silica particles composed of silica as a matrix and a fluorescent substance encapsulated therein, for example, can be produced by: preparing a solution in which inorganic semiconductor nanoparticles, a fluorescent substance such as an organic fluorescent dye, and a silica precursor such as tetraethoxysilane are dissolved; and then adding the thus prepared solution into a solution in which ethanol and ammonia are dissolved, thereby hydrolyzing the silica precursor.

On the other hand, fluorescent substance-integrated resin particles composed of a resin as a matrix and a fluorescent substance adsorbed onto the surface of the resin particles or encapsulated in the resin particles, can be produced by: preparing a solution of such a resin or a dispersion of resin nanoparticles in advance; and adding thereto inorganic semiconductor nanoparticles and a fluorescent substance such as an organic fluorescent dye, followed by stirring. Alternatively, the fluorescent substance-integrated resin particles can also be produced by adding a fluorescent dye to a solution of a resin raw material, and then allowing a polymerization reaction to proceed. For example, in cases where a thermosetting resin such as a melamine resin is used as a matrix resin, a reaction mixture containing: the raw material of the resin (a monomer, or an oligomer or prepolymer, for example, methylol melamine which is a condensation product of melamine and formaldehyde); an organic fluorescent dye; and preferably, a surfactant and a polymerization reaction accelerator (such as an acid) in addition; can be heated to allow a polymerization reaction to proceed by emulsion polymerization, thereby producing organic fluorescent dye-integrated resin particles. Further, in cases where a thermoplastic resin such as a styrene copolymer is used as a matrix resin, a reaction mixture containing: the raw material of the resin; an organic fluorescent dye (alternatively, a monomer to which the organic fluorescent dye has been bound in advance by a covalent bond or the like may also be used as the raw material monomer of the resin); and a polymerization initiator (such as benzoyl peroxide or azobisisobutyronitrile); can be heated to allow a polymerization reaction to proceed by radical polymerization or ion polymerization, thereby producing organic fluorescent dye-integrated resin particles.

Examples of the fluorescent substance to be integrated into the phosphor integrated dots include, in addition to the semiconductor nanoparticles and fluorescent dyes as described above, "long-afterglow phosphors" composed of Y₂O₃, Zn₂SiO₄ or the like as a matrix, and Mn²⁺, Eu³⁺ or the like as an activator.

The average particle size of the phosphor integrated dots (particularly, the fluorescent dye-integrated resin particles obtained by the production method as described above) is not particularly limited, as long as it is suitable for the immunostaining (or nucleic acid staining) of a pathological specimen. However, the average particle size is usually from 10 to 500 nm, and preferably from 50 to 200 nm, so that the particles can be easily detected as bright spots. Further, the phosphor integrated dots usually have a coefficient of variation, which indicates the variation in the particle size, of 20% or less, and preferably from 5 to 15%. The phosphor integrated dots which satisfy such conditions can be produced by adjusting the production conditions. For example, in the case of producing the phosphor integrated dots by emulsion polymerization, the average particle size thereof can be controlled by adjusting the amount of a surfactant to be added. In general, a relatively higher amount of a surfactant with respect to the amount of the raw material of the matrix of the phosphor integrated dots tends to result in a smaller particle size, and a relatively lower amount of the surfactant tends to result in a larger particle size.

The particle size of a phosphor integrated dot can be obtained by capturing an electron microscope image using a scanning electron microscope (SEM), measuring the sectional area of the phosphor integrated dot, and calculating the diameter of a circle corresponding to the sectional area, assuming the shape of the cross section to be a circle. The average particle size of a plurality of particles of phosphor integrated dots is determined by calculating the particle sizes of a sufficient number (such as 1,000 particles) of phosphor integrated dots as described above, and then calculating the arithmetic mean of the calculated particle sizes; and the coefficient of variation of the plurality of particles of phosphor integrated dots is calculated according to the equation: 100 × standard deviation of particle sizes /average particle size.

### < Structure of Immunostaining Agent >

The fluorescent nanoparticles to be contained in an immunostaining agent for fluorescent labeling of the target biological substance are preferably "phosphor integrated dots", as described above. In order to improve the efficiency of the fluorescent labeling, and to minimize the time required for the labeling, the prolonged duration of which leads to the degradation of fluorescence, it is preferred to use, as the immunostaining agent, a complex in which a primary antibody and a phosphor are linked indirectly, namely, linked by a bond other than a covalent bond, utilizing an antigen antibody reaction or an avidin-biotin reaction.

One example of the immunostaining agent in which a probe and a fluorescent nanoparticle are linked indirectly is: [primary antibody against target biological substance]... [antibody (secondary antibody) against primary antibody]-to-[fluorescent nanoparticle (phosphor integrated dot)]. In the above example, "..." represents a bond formed by an antigen antibody reaction. The mode of the bond represented by "-to-" is not particularly limited, and examples thereof include: a covalent bond, an ionic bond, a hydrogen bond, a coordinate bond, a physical adsorption, and a chemical adsorption, each of which may be formed via a linker molecule, as necessarily. For example, it is possible to use a silane coupling agent, which is a compound widely used for binding an inorganic substance with an organic substance. The silane coupling agent is a compound having an alkoxysilyl group which provides a silanol group upon hydrolysis at one end of the molecule, and having a functional group such as a carboxyl group, an amino group, an epoxy group, or an aldehyde group on the other end, and binds to an inorganic substance via the oxygen atom of the silanol group. Specific examples of the silane coupling agent include mercaptopropyltriethoxysilane, glycidoxypropyltriethoxysilane, aminopropyltriethoxysilane, and silane coupling agents having a polyethylene glycol chain (such as PEG-silane no. SIM 6492.7, manufactured by Gelest, Inc.). In the case of using a silane coupling agent, two or more types may be used in combination.

The reaction between the fluorescent nanoparticles and the silane coupling agent can be carried out using a known technique. For example, the resulting fluorescent substance-encapsulating silica nanoparticles are dispersed in pure water, followed by adding aminopropyltriethoxysilane thereto, and the mixture is allowed to react at room temperature for 12 hours. After the completion of the reaction, the resultant is subjected to centrifugation or filtration, to obtain fluorescent substance-encapsulating silica nanoparticles whose surfaces have been modified with aminopropyl groups. Subsequently, the amino group is allowed to react with a carboxyl group of an antibody, to bind the molecules of the antibody to the fluorescent substance-encapsulating silica nanoparticles via amide bonds. If necessary, a condensation agent such as EDC (1-Ethyl-3-[3-Dimethylaminopropyl]carbodiimide hydrochloride; manufactured by Pierce) can be used.

If necessary, it is possible to use a linker compound having a site capable of directly binding to a fluorescent substance-encapsulating silica nanoparticle modified with an organic molecule, and a site capable of binding to a molecular target substance. Specifically, when sulfo-SMCC (Sulfosuccinimidyl-4-[N-maleimidomethyl]cyclohexane-1-carboxylate; manufactured by Pierce) having both a site which selectively reacts with an amino group and a site which selectively reacts with a mercapto group is used, the amino group of the fluorescent substance-encapsulating silica nanoparticle modified with aminopropyltriethoxysilane can be bound to the mercapto group of an antibody, to provide fluorescent substance-encapsulating silica nanoparticles to which the molecules of the antibody are bound.

Binding of a biological substance recognition site (a site capable of specifically recognizing a biological substance, such as biotin, avidin, an antibody or the like) to a fluorescent substance-encapsulating polystyrene particle can be achieved the same procedure, even in the case of using a fluorescent dye as the fluorescent substance, or in the case using semiconductor nanoparticles. In other words, by impregnating polystyrene nanoparticles having a functional group such as an amino group with semiconductor nanoparticles or a fluorescent organic dye, it is possible to obtain fluorescent substance-integrated polystyrene particles having a functional group, and a subsequent use of EDC or sulfo-SMCC allows for obtaining fluorescent substance-integrated polystyrene particles to which the molecules of an antibody are bound.

Another example of the immunostaining agent in which a probe and a phosphor are linked indirectly is a complex composed of three molecules bound by the following binding mode: [primary antibody against target biological substance]... [antibody (secondary antibody) against primary antibody]-[biotin]/[avidin]-[phosphor (fluorescent nanoparticle)] (wherein "..." represents a bond formed by an antigen antibody reaction; "-" represents a covalent bond which may be formed via a linker molecule if necessary; and "/" represents a bond formed by an avidin-biotin reaction).

A secondary antibody-biotin conjugate (biotin-modified secondary antibody) can be produced using, for example, a commercially available biotin-labeling reagent (kit), based on a known technique capable of binding biotin to a desired antibody (protein). Alternatively, if a biotin-modified secondary antibody in which biotin has been bound to a desired antibody in advance is commercially available, such a product can be used as well.

Likewise, a fluorescent nanoparticle-avidin conjugate (avidin-modified phosphor) can be produced using, for example, a commercially available avidin-labeling reagent (kit), based on a known technique capable of binding avidin to a phosphor. The avidin to be used in this case may be of an improved type, such as streptavidin or NeutrAvidin, which exhibits a higher binding strength to biotin as compared to avidin.

Specific examples of the method of producing a phosphor-avidin conjugate include the followings. In cases where fluorescent nanoparticles are phosphor integrated dots containing a resin as a matrix, a functional group contained in the resin can be bound to a functional group contained in avidin (protein), via a linker molecule such as PEG having functional groups on both ends of the molecule, as necessary. For example, when the resin is a melamine resin, a functional group such as an amino group can be used; and when the resin is an acrylic resin, a styrene resin, or the like, a monomer having a functional group (such as an epoxy group) in its side chain can be copolymerized to utilize the functional group itself or a functional group converted from the functional group (for example, an amino group generated by a reaction with aqueous ammonia), or alternatively, such a functional group can be utilized to introduce another functional group. In cases where fluorescent nanoparticles are phosphor integrated dots containing silica as a matrix, or inorganic semiconductor nanoparticles, a desired functional group can be introduced by carrying out surface modification with a silane coupling agent. For example, the use of aminopropyltrimethoxysilane allows for introducing an amino group. On the other hand, a thiol group can be introduced into avidin by allowing, for example, N-succinimidyl S-acetylthioacetate (SATA) to react with the amino group of avidin. Subsequently, N-hydroxysuccinimide (NHS) ester reactive with an amino group, and a cross-linker reagent containing a polyethylene glycol (PEG) chain having a maleimide group reactive with a thiol group on each end thereof, can be used to link the phosphor having an amino group and avidin into which a thiol group is introduced.

A secondary antibody-to-fluorescent nanoparticle conjugate can be produced using, for example, a commercially available fluorescent labeling reagent (kit), based on a known technique capable of binding a desired fluorescent dye to a desired antibody (protein). Alternatively, if a secondary antibody-to-fluorescent nanoparticle conjugate in which a desired antibody has been bound to a desired fluorescent nanoparticle in advance is commercially available, such a product can be used as well.

### - Method of Staining Tissue Section -

### (Immunostaining Method)

A description will be given below regarding one example of immunostaining using fluorescent nanoparticles, which can be used in the present invention for quantifying the expression level of a cancer-associated protein in a tumor cell and/or that of a protein in an immune cell, using a specimen derived from a tumor tissue of a human or a non-human, typically, a tissue section (sample slide), and further, for quantifying a cytokine and/or a membrane vesicle-associated protein contained in the specimen, as necessary.

The methods of preparing a tissue section (also simply referred to as a "section" in the present specification, and used herein as a term encompassing sections such as pathological sections) and a sample slide on which the tissue section is placed, are not particularly limited, and those prepared by known methods can be used.

### (1. Sample Preparation Step)

### (1-1. Deparaffinization Treatment)

The subject section is immersed in a container filled with xylene to remove paraffin. The temperature in this process is not particularly limited, and may be room temperature. The section is preferably immersed for an immersion time of three minutes or more and 30 minutes or less. If necessary, xylene may be replaced during the immersion.

Subsequently, the section is immersed in a container filled with ethanol to remove xylene. The temperature in this process is not particularly limited, and may be room temperature. The immersion time is preferably three minutes or more and 30 minutes or less. If necessary, ethanol may be replaced during the immersion.

The section is then immersed in a container filled with water to remove ethanol. The temperature in this process is not particularly limited, and may be room temperature. The immersion time is preferably three minutes or more and 30 minutes or less. If necessary, water may be replaced during the immersion.

### (1-2. Activation Treatment)

The activation treatment of a target biological substance is carried out in accordance with a known method. The conditions for activation are not particularly defined, and a 0.01 M citric acid buffer solution (pH 6.0), a 1 mM EDTA solution (pH 8.0), 5% urea, a 0.1 M tris-hydrochloric acid buffer solution, or the like can be used as an activation liquid. An autoclave, a microwave oven, a pressure cooker, a water bath or the like can be used as a heating apparatus. The temperature is not particularly limited, and may be room temperature. The heating can be performed at a temperature of from 50 to 130°C, for a period of time of from five to 30 minutes.

Subsequently, the section after being subjected to the activation treatment is immersed in a container filled with PBS to carry out washing. The temperature in this process is not particularly limited, and may be room temperature. The immersion time is preferably three minutes or more and 30 minutes or less. If necessary, PBS may be replaced during the immersion.

### (2. Immunostaining Step)

In the immunostaining step, fluorescent nanoparticles having a site capable of binding to the target biological substance directly or indirectly are dispersed in a diluent for fluorescent nanoparticles, and the resulting dispersion is placed on the tissue section to allow the fluorescent nanoparticles to react with the target biological substance, in order to carry out the staining of the biological substance. The immunofluorescent staining solution or the diluent for fluorescent nanoparticles for preparing the same, and other components, to be used in the immunostaining step are as described above, and may be prepared in advance before carrying out this step.

For example, in cases where the immunostaining agent is a complex having the structure: [primary antibody (probe)]... [secondary antibody]-[biotin]/[avidin]-[fluorescent nanoparticle (phosphor integrated dot or the like)] (wherein "..." represents a bond formed by an antigen antibody reaction; "-" represents a covalent bond which may be formed via a linker molecule if necessary; and "/" represents a bond formed by an avidin-biotin reaction), the immunostaining can be achieved by: first carrying out a treatment (primary reaction treatment) in which a pathological specimen is immersed in a solution of a primary antibody; then carrying out a treatment (secondary reaction treatment) in which the pathological specimen is immersed in a solution of a secondary antibody-biotin conjugate; and finally carrying out a treatment (fluorescent labeling treatment) in which the tissue section, which is the pathological specimen, is immersed in a solution (immunofluorescent staining solution) obtained by dispersing avidin-fluorescent nanoparticles in the diluent for fluorescent nanoparticles according to the present invention.

The conditions for carrying out the immunostaining step, for example, the temperature and the immersion time, when the tissue section as the pathological specimen is immersed in a predetermined solution (reagent) in each of the primary reaction treatment, the secondary reaction treatment and the fluorescent labeling treatment, can be adjusted as appropriate in accordance with a conventional immunostaining method, so as to obtain appropriate signals. The temperature in this process is not particularly limited, and may be room temperature. The reaction is preferably carried out for 30 minutes or more and 24 hours or less.

Before carrying out the primary reaction treatment as described above, it is preferred to add a known blocking agent such as BSA-containing PBS or a surfactant such as Tween 20, dropwise. In the present invention, even in cases where such a treatment of adding a blocking agent (blocking treatment) before the primary reaction treatment is carried out, it is possible to obtain the effects of the present invention, such as reducing the background noise etc., by incorporating specific two types of proteins in predetermined amounts into the immunofluorescent staining solution (or the diluent for fluorescent nanoparticles for preparing the same) to be used in the fluorescent labeling treatment.

### (3. Sample Post-treatment Step)

The pathological specimen which has been subjected to the immunostaining step is preferably subjected treatments such as immobilization and dehydration, clearing, and sealing, so that the tissue section is made suitable for observation.

The immobilization and dehydration treatment can be achieved by immersing the pathological specimen into an immobilization treatment liquid (a crosslinking agent such as formalin, paraformaldehyde, glutaraldehyde, acetone, ethanol, or methanol). The clearing treatment can be achieved by immersing the pathological specimen which has been subjected to the immobilization and dehydration treatment into a clearing liquid (xylene or the like). The sealing treatment can be achieved by immersing the pathological specimen which has been subjected to the clearing treatment into a sealing liquid. The conditions for carrying out these treatments, for example, the temperature and the immersion time when the pathological specimen is immersed in a predetermined treatment liquid in each of the treatments, can be adjusted as appropriate in accordance with a conventional immunostaining method, so as to obtain an appropriate signal.

### (4. Optional Step)

In the present invention, a staining step for morphological observation can be included, if necessary, so that the morphology of cells, tissue, an organ or the like can be observed in the bright field. The staining step for morphological observation can be carried out in accordance with a conventional method. For the morphological observation of a tissue sample, staining using eosin is typically employed, by which cytoplasm, interstitium, various types of fibers, erythrocytes, and keratinocytes are stained red to dark red. Further, staining using hematoxylin is also typically employed, by which cell nuclei, calcareous parts, cartilage tissue, bacteria, and mucus are stained livid to light blue (a method in which these two types of staining are carried out simultaneously is known as hematoxylin-eosin staining (HE staining). In the case of including the staining step for morphological observation, the step may be carried out after the immunostaining step, or may be carried out before the immunostaining step.

### (5. Evaluation Step)

### (5-1. Observation and Image Capture)

In the observation and image capture step, the pathological specimen is irradiated with the respective types of excitation light corresponding to the respective types of phosphors fluorescently labeling the target biological substance used in the immunostaining step, in the same visual field of a microscope at a desired magnification, and the observation and image capture of the fluorescence emitted from the respective types of phosphors are carried out, to obtain immunostained images. The irradiation of each excitation light can be performed, for example, using a laser beam source included in a fluorescence microscope, and an optical filter for excitation light which selectively transmits a predetermined wavelength, as necessary. The capture of immunostained images can be carried out, for example, by a digital camera included in the fluorescence microscope. If necessary, an optical filter for fluorescence which selectively transmits a predetermined wavelength can be used, when capturing immunostained images, to capture immunostained images including only the desired fluorescence, from which images undesired fluorescence, excitation light that becomes noise, and other types of light are excluded.

### (5-2. Image Processing and Signal Measurement)

In the image processing and measurement step, the signals of the fluorescent labels corresponding to the target biological substance are measured in the immunostained images captured for the target biological substance, based on image processing, and the signals of the fluorescent labels corresponding to the target biological substance which are present within the region of the cell membrane are identified. The signals of the fluorescent labels are preferably measured in terms of the number of bright spots of fluorescence.

Examples of software which can be used for the image processing include "ImageJ" (open source). The use of such image processing software allows for carrying out the processing to extract bright spots having a predetermined wavelength (color) from the immunostained images and to calculate the total sum of the brightness, and to count the number of bright spots having a brightness equal to or higher than a predetermined brightness, particularly, the processing to carry out the above described embodiments, quickly and in a semi-automatic manner.

Further, since one bright spot is derived from one fluorescent nanoparticle, the bright spots have a constant size and can be detected by a microscope observation. A bright spot having a signal higher than a certain value (for example; the mean value of the fluorescent nanoparticles to be observed) is determined as an aggregated bright spot. The aggregated bright spots can be discriminated from the bright spots quickly and semi-automatically using the software.

### (Nucleic Acid Staining)

In the present invention, in the case of quantifying a nucleic acid (DNA or RNA-related substance), namely, a target nucleic acid present in a specimen derived from tumor tissue of a human or a non-human, or in a specimen of extracellular vesicles, lymph node, or a body fluid such as blood or saliva, fluorescent nanoparticles which are the same as those used for the immunostaining described above can be used to specifically stain the target nucleic acid, in accordance with the FISH method. In other words, nucleic acid staining which can be carried out in the present invention is a method of staining the target nucleic acid, using a solution for nucleic acid staining, which contains the above described diluent for fluorescent nanoparticles, a probe, and fluorescent nanoparticles capable of binding to, or bound to, the probe.

The target nucleic acid may be a DNA such as a chromosome (region encoding a specific gene), or an RNA such as an mRNA, tRNA, miRNA, siRNA, or non-cording-RNA.

### (Target Nucleic Acid)

As the target nucleic acid, a desired nucleic acid can be selected depending on the object of the invention, namely, depending on the disease for which information for diagnosis or treatment is intended to obtain. For example, a chromosome containing a gene (the portion of the gene) encoding a biomolecule (protein) specifically expressed in tumor cells or immune cells, which is related to cancer, an immune system-related disease or the like may be taken as the target acetic acid, or alternatively, any other chromosomal or non-chromosomal nucleic acid (such as one liberated in an extracellular vesicle, lymph node, blood, body fluid or the like) may be taken as the target nucleic acid. Further, the nucleic acid may be a DNA such as a chromosome, or an RNA such as an mRNA, tRNA, miRNA, siRNA, or non-cording-RNA.

### (Probe)

The probe is a nucleic acid molecule having a sequence (probe sequence) including a part or whole of the sequence of the target nucleic acid (DNA or RNA) as described above. The nucleic acid molecule as the probe may be any nucleic acid molecule capable of forming a strand complementary to the target nucleic acid, namely, any nucleic acid molecule having a base sequence complementary to the target nucleic acid, and may be a DNA or an RNA. Further, the nucleic acid molecule may be: a nucleic acid composed of naturally-occurring bases which are the same as the target nucleic acid; an artificial nucleic acid such as PNA, LNA (or BNA: Bridged Nucleic Acid) or the like; or a nucleic acid molecule composed of a naturally-occurring nucleic acid linked to an artificial nucleic acid.

### (Preparation of Probe)

A probe for the target nucleic acid can be prepared in accordance with a known method, and can be obtained as a commercially available product. The base length, base sequence, and GC content of the probe can be adjusted, so that the conditions for hybridization have an appropriate stringency.

### (Binding of Probe to Fluorescent Nanoparticle)

Binding of a probe to a fluorescent nanoparticle can be achieved via any of various types of bonds without particular limitation, as long as nucleic acid staining (such as FISH) can be carried out without problems. The binding of the probe to the fluorescent nanoparticle may be achieved by either: a method in which the fluorescent nanoparticle is directly bound to the probe; or a method in which the fluorescent nanoparticle is indirectly bound to the probe via a bond between biomolecules.

### EXAMPLES

### [Preparation Example 1] Step of Preparing Red PID Staining Agent

### (Preparation of Biotin-modified Anti-rabbit IgG Antibody)

A quantity of 50 µg of an anti-rabbit IgG antibody as a secondary antibody was dissolved in a 50 mM Tris solution. To the resulting solution, a DTT (dithiothreitol) solution was added to a final concentration of 3 mM, followed by mixing, and the mixture was allowed to react at 37°C for 30 minutes. Subsequently, the reaction solution was allowed to pass through a desalting column "Zeba Desalt Spin Column" (Cat. #: 89882; manufactured by Thermo Fisher Scientific Inc.), to purify the secondary antibody which had been reduced with DTT. A quantity of 200 µL of the total amount of the purified antibody was dissolved in a 50 mM Tris solution, to prepare an antibody solution. Meanwhile, a linker reagent "Maleimide-PEG2-Biotin" (product number: 21901; manufactured by Thermo Fisher Scientific Inc.) was adjusted to a concentration of 0.4 mM with DMSO. A quantity of 8.5 µL of the thus prepared linker reagent solution was added to the antibody solution, followed by mixing. The mixture was allowed to react at 37°C for 30 minutes to bind biotin to the anti-rabbit IgG antibody via a PEG chain. The resulting reaction solution was purified by filtration through a desalting column. The absorbance of the desalted reaction solution was measured at a wavelength of 300 nm using a spectrophotometer ("F-7000" manufactured by Hitachi Ltd.) to calculate the concentration of the protein (biotin-modified secondary antibody) in the reaction solution. Using a 50 mM Tris solution, the concentration of the biotin-modified secondary antibody was adjusted to 250 µg/mL, and the thus prepared solution was used as a solution of the biotin-modified secondary antibody.

### (Preparation of Streptavidin-bound Texas Red Integrated Melamine Resin Particles)

A quantity of 2.5 mg of Texas Red dye molecules "Sulforhodamine 101" (manufactured by Sigma-Aldrich Co. LLC.) was dissolved in 22.5 mL of pure water, and the resulting solution was then stirred by a hot stirrer for 20 minutes, while maintaining the temperature of the solution at 70°C. To the stirred solution, 1.5 g of a melamine resin "NIKALAK MX-035" (manufactured by Nippon Carbide Industries Co., Inc.) was added, and the mixture was further heated and stirred for five minutes under the same conditions. To the stirred solution, 100 µL of formic acid was added, and the mixture was stirred for 20 minutes while maintaining the temperature of the solution at 60°C, and the resulting solution was allowed to cool to room temperature. The cooled solution was dispensed into a plurality of centrifugation tubes, and then centrifuged at 12,000 rpm for 20 minutes to precipitate Texas Red integrated melamine resin particles contained in the solution as a mixture. Each supernatant was removed, and the precipitated particles were washed with Ethanol and water. An SEM observation was carried out for 1,000 resulting nanoparticles to measure the average particle size as described above, and the average particle size of the particles was determined to be 152 nm. The thus prepared Texas Red integrated melamine resin particles were surface modified according to the following procedure, and the resulting particles were used as phosphor integrated dots (PIDs) in Examples 1 to 3.

A quantity of 0.1 mg of the thus obtained particles was dispersed in 1.5 mL of EtOH, followed by adding 2 µL of aminepropyltrimethoxysilane "LS-3150" (manufactured by Shin-Etsu Chemical Co., Ltd.) thereto, and the resultant was allowed to react for 8 hours to carry out a surface amination treatment.

Subsequently, PBS (phosphate buffered physiological saline) containing 2 mM EDTA (ethylenediaminetetraacetic acid) was used to prepare a solution of the particles which had been subjected to the surface amination treatment, having a concentration of 3 nM, and the resulting solution was mixed with SM (PEG) 12 (succinimidyl-[(N-maleimidopropionamido)-dodecaethyleneglycol] ester; manufactured by Thermo Fisher Scientific Inc.) to a final concentration of 10 mM, followed by a reaction for one hour. The thus obtained mixed liquid was centrifuged at 10,000 G for 20 minutes, and the supernatant was removed. PBS containing 2 mM EDTA was then added thereto to disperse the resulting precipitates, followed by another centrifugation. Washing by the same procedure was carried out three times, to obtain phosphor integrated melamine particles having a terminal-connected maleimide groups.

Meanwhile, streptavidin (manufactured by Wako Pure Chemical Corporation) was subjected to a thiol group-addition treatment using N-succinimidyl S-acetylthioacetate (SATA), and the resultant was filtered through a gel filtration column, to obtain a solution of streptavidin capable of binding to phosphor integrated melamine particles.

The above described phosphor integrated melamine particles and streptavidin were mixed in PBS containing 2 mM EDTA, and the mixture was allowed to react at room temperature for one hour. Thereafter, 10 mM mercaptoethanol was added to terminate the reaction. After concentrating the resulting solution with a centrifugal filter, unreacted streptavidin and the like were removed using a gel filtration column for purification, to prepare streptavidin-bound phosphor integrated melamine particles.

### [Preparation Example 2] Step of Preparing Green PID Staining Agent

FITC dye-integrated melamine resin nanoparticles having an average particle size of 159 nm were prepared, in accordance with the above described procedure (Preparation of Streptavidin-bound Texas Red Integrated Melamine Resin Particles), and using FITC instead of the Texas Red dye molecules "Sulforhodamine 101" (manufactured by Sigma-Aldrich Co. LLC.). The surface modification of the resulting particles was carried out in accordance with the above described procedure, using an anti-CD8 rabbit monoclonal antibody "SP16" instead of streptavidin, to prepare antibody-bound FITC integrated melamine resin particles, which were used as phosphor integrated dots (PIDs) in Example 3.

### [Example 1] Evaluation of Expression Level of PD-L1

### Sample Preparation Step

### (Sample Pre-treatment)

Lung tissue array slides "LC241b" manufactured by US BIOMAX INC. Inc. (glass slides on each of which the total of 24 pieces of sections are placed, including two pieces of tumor tissue sections and two pieces of normal tissue sections derived from each of six patients, and one piece of a section of a tissue marker) were purchased. After subjecting the samples to deparaffinization treatment, the samples were subjected to a displacement washing with water. An antigen activation treatment was carried out by subjecting the washed tissue array slides to an autoclave treatment in a 10 mM citric acid buffer solution (pH 6.0) at 121°C for 15 minutes. The tissue array slides after being subjected to the antigen activation treatment were washed with PBS, and the blocking treatment of the washed tissue array slides was carried out for one hour, using PBS containing 1% BSA.

### (Primary Reaction Treatment of Immunostaining)

Using PBS containing 1W/W% BSA, a primary reaction treatment liquid containing an anti-PD-L1 rabbit monoclonal antibody (clone "SP142"; manufactured by Spring Bioscience Corporation (SBS)) at a concentration of 0.05 nM was prepared, to be used in the primary reaction treatment for the first immunostaining of the target biological substance, PD-L1. The samples prepared in the sample pre-treatment step were immersed in the thus prepared primary reaction treatment liquid, and allowed to react at 4°C overnight.

### (Secondary Reaction Treatment of Immunostaining)

The solution of the biotin-modified anti-rabbit IgG antibody prepared in the above described section of "Preparation of PID Staining Agent" was further diluted to a concentration of 6 µg/mL, using PBS containing 1W/W% BSA, to prepare a secondary reaction treatment liquid. The samples after being subjected to the primary reaction treatment were washed with PBS, and then immersed in the thus prepared secondary reaction treatment liquid, and allowed to react at room temperature for 30 minutes.

### (Labeling Treatment-1 of Immunostaining: DAB Labeling)

After washing the samples which had been subjected to the secondary reaction treatment, the samples were immersed in streptavidin-HRP (21130; manufactured by Thermo-Fisher), and allowed to react at room temperature for 60 minutes. Subsequently, the samples were washed with PBS, and then immersed in a DAB (3, 3'-Diaminobenzidine) solution for one minute.

### (Labeling Treatment-2 of Immunostaining: PID Fluorescent Labeling)

Using a diluent for fluorescent nanoparticles in which the contents of casein (composition = α-casein (c6780; manufactured by Sigma-Aldrich Co. LLC.): 50 W/W%, β-casein (c6905; manufactured by Sigma-Aldrich Co. LLC.): 50W/W%) and BSA were adjusted to 1% and 3%, respectively, the streptavidin-modified Texas Red dye-integrated melamine resin particles prepared in the above described section of "Preparation of PID Staining Agent" were diluted to a concentration of 0.02 nM, to prepare a fluorescent labeling reaction treatment liquid. The samples which had been subjected to the secondary reaction treatment were immersed in the fluorescent labeling treatment liquid, and allowed to react at room temperature for three hours.

The DAB labeling treatment and the PID fluorescent labeling treatment were carried out for separate lung tissue array slides (each including the same set of tissue sections, and the treatments were respectively carried out for adjacent sections). The following data regarding the results of the expression rate of DAB and the number of fluorescent bright spots, shown in Table 1, are data each obtained from the tissue of the same corresponding patient, and each represents the mean value of the numerical values of the two pieces of tissue sections.

### (Sample Post-treatment)

The samples which had been immunostained were subjected to an immobilization and dehydration treatment, in which an operation of immersing the samples in pure Ethanol for five minutes was repeated four times. Subsequently, the samples were subjected to a clearing treatment, in which an operation of immersing the samples in xylene for five minutes was repeated four times. Finally, a sealing treatment was carried out in which a sealant "Entellan New" (manufactured by Merck KGaA) was applied on the samples and coverslips were placed thereover, and the resultants were used as samples for use in observation.

### Evaluation Step

### (Expression rate of PD-Ll)

The samples which had been subjected to the DAB labeling treatment were observed by a microscope, and the expression rate of PD-L1 was calculated as the ratio of the number of stained cells with respect to the number of observed cells, in accordance with the description in: N Engl J Med. 2012 June 28; 366 (26): 2443-2454 (non-patent document 1).

### (Number of Fluorescent Bright Spots)

A fluorescence microscope "BX-53" (manufactured by Olympus Corporation) was used for the observation of the fluorescence emission, and a digital camera for a microscope, "DP73" (manufactured by Olympus Corporation) attached to the fluorescence microscope was used for capturing immunostained images (400-fold).

First, an excitation light corresponding to the Texas Red dye used for the fluorescent labeling of the target biological substance PD-L1 was irradiated to each sample to allow fluorescence emission to occur, and an immunostained image of the sample in that state was captured. At this time, the wavelength of the excitation light was set within the range of from 575 to 600 nm, using an optical filter for excitation light included in the fluorescence microscope, and the wavelength of the fluorescence to be observed was adjusted within the range of from 612 to 692 nm, using an optical filter for fluorescence. The intensity of the excitation light during the observation and image capture by the fluorescence microscope was adjusted such that the irradiation energy in the vicinity of the center of the visual field was 900 W/cm². The exposure time during the image capture was adjusted within a range such that the brightness of the image to be captured was not saturated, such as, for example, to 4000 µ seconds.

Immunostained images were captured as described above in the same visual field, and then the same operation was repeated, changing the visual field each time, to obtain images captured in the total of five different visual fields (the first to the fifth visual fields) per one sample.

Image processing software "ImageJ" (open source) was used for the image processing in this step.

Among the bright spots indicating the Texas Red dye-integrated melamine resin particles fluorescently labeling the molecules of PD-L1 in each of the immunostained images, the number of bright spots having a brightness equal to or higher than a predetermined value was counted. The thus counted number was used as an index for evaluating the immune reaction.

The results of Example 1 are shown in Table 1. It has been shown that there is a correlation between the evaluation of the expression rate of PD-L1 as measured by conventional DAB labeling, and the evaluation of the number of particles per cell as measured by the PID-labeling of the present invention. Thus, it can be seen that the use of the PID method allows for quantifying the expression level of a cancer-associated protein expressed in a cancer cell with a high accuracy, and for obtaining information which can be used for the diagnosis or treatment of cancer.

**[Table 1]**

| Sample slide | Associated information (Lung cancer stage) | Number per cell of particles indicating PD-L1 | PD-L1 expression rate (%) |
|---|---|---|---|
| A | II | 6 | 5 |
| B | III | 85 | 80 |
| C | I | 22 | 15 |
| D | II | 2 | 0 |
| E | I | 28 | 22 |
| F | III | 65 | 55 |
| G | I | 4 | 2 |
| H | I | 4 | 0 |
| I | II | 81 | 77 |
| J | II | 6 | 1 |

### [Example 2] Evaluation of Expression Level of CD8

Staining and evaluation were carried out in the same manner as in Example 1, except that the autoclave treatment was carried out for five minutes, and an anti-CD8 rabbit monoclonal antibody "SP16" at a concentration of 1 µg/mL (Code GTX79429; manufactured by Genetex Inc.) was used instead of the anti-PD-L1 rabbit monoclonal antibody "SP142", in the sample pre-treatment step.

The results of Example 2 are shown in Table 2. It has been shown that there is a correlation between the evaluation of the expression rate of CD8 as measured by conventional DAB labeling, and the evaluation of the number of particles per cell as measured by the PID-labeling of the present invention. Thus, it can be seen that the use of the PID method allows for quantifying the expression level of a predetermined protein expressed in an immune cell with a high accuracy, and for obtaining information which can be used for the diagnosis or treatment of an immune system-related disease (including cancer).

**[Table 2]**

| Sample slide | Associated information (Lung cancer stage) | Number per cell of particles indicating CD8 | CD8 expression rate (%) |
|---|---|---|---|
| A | II | 25 | 10 |
| B | III | 40 | 5 |
| C | I | 10 | 8 |
| D | II | 78 | 20 |
| E | I | 40 | 15 |
| F | III | 41 | 12 |
| G | I | 55 | 26 |
| H | I | 35 | 10 |
| I | II | 72 | 24 |
| J | II | 29 | 17 |

### [Example 3] Evaluation of Distance between CD8-expressing T cell and PD-L1-expressing Cancer Cell

Sample slides which had been subjected to the red PID staining for PD-L1 in cancer cells in accordance with the procedure described in the section of "Labeling Treatment-2 of Immunostaining: PID Fluorescent Labeling" were immersed in a 0.02 nM solution of the antibody-bound FITC dye-integrated melamine resin particles obtained in Preparation Example 2. The resulting slides were allowed to react at room temperature for three hours, to carry out green PID staining for CD8 in immune cells. Subsequently, the observation of the bright spots was carried out in the same manner as the description following the section of (Sample Post-treatment), and the distance between red bright spots and green bright spots was measured.

The results of the distance between cells are shown in Table 3. The results have revealed that the distance between a PD-L1 - expressing cancer cell and a CD8-expressing T cell (activated killer T cell) can be quantified as the distance between bright spots. Thus, according to the embodiment in which the expression level of a predetermined protein is quantified based on the PID method, it is possible to quantify the expression level of a predetermined protein in a cancer cell and that in an immune cell, as well as to measure the distance between these cells, thereby allowing for obtaining complex information.

The immune score test and the MSI test are both standard tests, and were carried out by an outsourcing service. The results thereof are also shown in Table 3. Upon comparing the results of the immune score test and the MSI test obtained in Example 1 and Example 2, it has been revealed that the results of the bright spot measurement by the present invention are correlated with those of the expression rate, as described above, and in addition, correlated to a certain extent with the results of the immune score (the above described 8 items) and the MSI test. Further, the results of the measurement of the distance between bright spots by the present invention show a higher level of correlation with the results of the immune score (the above described 8 items) and the MSI test. Accordingly, it is assumed that the present invention, which allows for quantifying the expression level of a predetermined protein and the distance between cells based on the PID method, can further be applied to various types of immune checkpoint proteins, and enables to obtain information which can be used for the diagnosis of cancer and which is more complex than that obtainable by conventional methods.

**[Table 3]**

| Sample slide | Distance between CD8-expressing T cell and PD-L1-expressing cancer cell (µm) | Immune score (8 items) | MSI Test |
|---|---|---|---|
| A | 150 | 12 | H |
| B | 200 | 5 | L |
| C | 250 | 1 | MSS |
| D | 20 | 14 | H |
| E | 280 | 8 | L |
| F | 40 | 16 | L |
| G | 5 | 2 | H |
| H | 180 | 8 | MSS |
| I | 0 | 22 | H |
| J | 350 | 14 | H |

### [Reference Example1] Evaluation of Expression Level of miR197

The staining and the evaluation of miR197, which is a miRNA expressed in tumor cells, were carried out, using lung tissue array slides "LC241b" which had been subjected to the pre-treatment in the same manner as in Example 1.

### (Primary Reaction Treatment of Nucleic Acid Staining)

A primary reaction treatment liquid containing fluorescein-labeled fluorescent probe (hsa-miR-197-3p, 611339-310; manufactured by EXIQON) at a concentration of 100 ng/µL was prepared, using IQFISH FFPE Hybridization Buffer (manufactured by Agilent Technologies, Inc.), and used in the primary reaction treatment for the first nucleic acid staining of the target biological substance miR197. The samples prepared in the sample pre-treatment step were immersed in the thus prepared in primary reaction treatment liquid, and allowed to react at 80°C for 10 minutes, followed by a further reaction at 4°C overnight.

### (Secondary Reaction Treatment of Nucleic Acid Staining)

A solution of BA-0601 manufactured by Vector, as a biotin-labeled anti-fluorescein antibody, was further diluted to a concentration of 6 µg/mL, using PBS containing 1 W/W% BSA, to prepare a secondary reaction treatment liquid. The samples after being subjected to the primary reaction treatment were washed with PBS, and then immersed in the thus prepared secondary reaction treatment liquid, and allowed to react at room temperature for 30 minutes.

### (Amplification Reaction Treatment of Nucleic Acid Staining)

The samples were immersed in 40 µL of a streptavidin/ HRP primary enzyme reagent of Genpoint (trademark; Dako), and allowed to react at room temperature for 15 minutes, followed by washing with TBST. The samples were further allowed to react at room temperature for 15 minutes, using a biotinylated tyramide solution.

### (Labeling Treatment-1 of Immunostaining: DAB Labeling)

The samples which had been subjected to the amplification reaction treatment were washed with TBS, and then immersed in 40 µL of a streptavidin/ HRP secondary enzyme reagent of Genpoint (trademark; Dako). After allowing the samples to react at room temperature for 15 minutes, the samples were washed with TBST, immersed in a DAB (3, 3'-Diaminobenzidine) solution for one minute, and washed with water.

### (Labeling Treatment-2 of Nucleic Acid Staining: Red PID Fluorescent Labeling)

Using a diluent for phosphor integrated dots in which the contents of casein (composition = α-casein (c6780; manufactured by Sigma-Aldrich Co. LLC.): 50 W/W%, β-casein (c6905; manufactured by Sigma-Aldrich Co. LLC.): 50W/W%) and BSA were adjusted to 1% and 3%, respectively, the streptavidin-bound Texas Red dye-integrated melamine resin particles prepared in the Preparation Example 1 were diluted to a concentration of 0.1 nM, to prepare a fluorescent labeling reaction treatment liquid. The samples which had been subjected to the secondary reaction treatment were immersed in the thus prepared fluorescent labeling treatment liquid, and allowed to react at room temperature for one hour, followed by washing with PBS.

The samples after being subjected to the DAB labeling treatment and the PID fluorescent labeling treatment were post-treated and evaluated in the same manner as in Example 1. The results are shown in Table 4.

**[Table 4]**

| Sample slide | Associated information (Lung cancer stage) | Number per cell of particles indicating miR197 | miR197 expression rate (%) |
|---|---|---|---|
| A | I | 15 | 15 |
| B | III | 8 | 40 |
| c | I | 22 | 16 |
| D | I | 10 | 0 |
| E | I | 28 | 25 |
| F | II | 39 | 54 |
| G | I | 21 | 10 |
| H | I | 28 | 5 |
| I | III | 8 | 20 |
| J | I | 12 | 35 |

By performing the nucleic acid staining as described above, the expression level of a cancer cell gene can be quantified. Even if the quantification of a miRNA, as a nucleic acid, contained in a cancer (tumor) cell is carried out alone, as described in the Reference Example 1, it is possible to obtain a certain level of information for diagnosis or treatment.

Further, a phenomenon has been recently reported that a miRNA, such as miR181c, migrates between the cells using an exosome as a carrier (Nat Commun. 2015 Apr 1; 6: 6716. doi: 10.1038/ ncomms 7716). Accordingly, it is thought that the use of the above described technique enables to obtain information regarding the dynamic migration of various types of miRNAs.

### [Example 4] Evaluation of Distance between miR197-expressing Cancer Cell and CD8-expressing T cell

Sample slides which had been subjected to the red PID staining for miR197 in cancer cells in accordance with the procedure described in the section of "Labeling Treatment of Nucleic Acid Staining: Red PID Fluorescent Labeling" were allowed to react with a solution of the antibody-bound FITC integrated melamine resin particles prepared in Preparation Example 2, in the same manner as in Example 3, to carry out green PID staining for CD8 in immune cells. Subsequently, the observation of the bright spots was carried out in the same manner as the description following the section of Sample Post-treatment in Example 1, and the distance between red bright spots and green bright spots was measured.

As a result, it has been revealed that the distance between a miRNA197-expressing cancer cell and a CD8-expressing T cell (activated killer T cell) can be quantified as the distance between bright spots, in the same manner as in Example 3. It can be said that an embodiment in which the quantification of the expression level of a protein in an immune cell (Example 2), the quantification of the expression level of a nucleic acid (miRNA) in a cancer cell (Reference Example 1), and further, the measurement of the distance between the cells expressing the protein and the nucleic acid (Reference Example 2) are carried out as described above, is a preferred embodiment of the present invention. In addition, it is also possible to clarify the distance between a cell expressing a protein specific to cancer cells and a cell expressing a nucleic acid specific to immune cells, in the same manner.

### [Example 5] Patient-derived Tumor-transplanted Mouse

Tissue specimen collected from one lung cancer patient was purchased from Sofia Bio, to prepare an Immune-PDX model mouse. Cancer tissue having a size of 2 mm square was transplanted subcutaneously, and the tissue of the cancer which had grown to a size of about 300 mm³ was collected, one month after the transplantation, to prepare a FFPE tissue slide.

Staining was carried out in the same manner as in Examples 1, 2 and 3, and the following results were obtained. It can be seen from these results that, even in an alternative test system using a patient-derived tumor-transplanted model mouse, the use of the PID method enables to quantify the expression level of a predetermined protein expressed in an immune cell with a high accuracy, and to obtain information which can be used for the diagnosis or treatment of an immune system-related disease (including cancer).

**[Table 5]**

| Number per cell of particles indicating PD-L1 | PD-L1 expression rate | Number per cell of particles indicating CD8 | CD8 expression rate | Distance |
|---|---|---|---|---|
| 40 | 35% | 25 | 5% | 200 nm |

### [Example 6] Cultured Cancer Cell-transplanted Mouse

A cancer FFPE tissue slide was prepared in the same manner as in Example 4, except that tumor tissue collected from a naturally induced tumor-bearing mouse produced by introducing a carcinogenic substance (3-methylcholanthrene (3-MCA)) into a breast thereof was used for transplantation.

The slide prepared as described above was stained in the same manner as in Examples 1, 2 and 3, except that an anti-mouse PD-L1 rabbit antibody (product number: PB9994, manufactured by Boster Immunoleader) was used instead of the anti-human PD-L1 rabbit monoclonal antibody (clone "SP142") as the primary antibody, and an anti-mouse CD8 rabbit antibody, "product number: STJ20180, manufactured by St Jones lab" was used instead of the anti-human CD8 rabbit monoclonal antibody "SP16".

As a result, the following results were obtained. It can be seen from these results that, even in an alternative test system using a patient-derived tumor-transplanted model mouse, the use of the PID method enables to quantify the expression level of a predetermined protein expressed in an immune cell with a high accuracy, and to obtain information which can be used for the diagnosis or treatment of an immune system-related disease (including cancer).

**[Table 6]**

| Number per cell of particles indicating PD-L1 | PD-L1 expression rate | Number per cell of particles indicating CD8 | CD8 expression rate | Distance |
|---|---|---|---|---|
| 4 | 12% | 11 | 10% | 40 nm |

## Claims

1. A method of obtaining information for diagnosis or treatment, the method comprising the step of quantifying the expression level of a cancer-associated protein in a tumor cell, using a specimen derived from tumor tissue of a human or a non-human.

2. A method of obtaining information for diagnosis or treatment, the method comprising the step of quantifying the expression level of a protein in an immune cell, using a specimen derived from tumor tissue of a human or a non-human.

3. The method of obtaining information according to claim 1 or 2, wherein the information for diagnosis or treatment is related to cancer or an immune system-related disease.

4. The method of obtaining information according to any one of claims 1 to 3, the method further comprising the step of measuring the distance between the tumor cell and the immune cell, or the distance between immune cells which interact with each other, in the specimen derived from tumor tissue of a human or a non-human.

5. The method of obtaining information according to any one of claims 1 to 4, the method further comprising the step of quantifying a nucleic acid, a cytokine or a membrane vesicle-associated protein, present in the specimen derived from tumor tissue of a human or a non-human, or in a specimen of extracellular vesicles, lymph node, blood or body fluid, of a human or a non-human.

6. The method of obtaining information according to claim 5, the method comprising the step of quantifying, as the nucleic acid present in the specimen derived from tumor tissue of a human or a non-human, a miRNA contained in an immune cell or a miRNA contained in a tumor cell, which miRNA regulates the expression level of the cancer-associated protein in the tumor cell or the protein in the immune cell.

7. The method of obtaining information according to any one of claims 1 to 6, the method further comprising the step of measuring an immune score and/or carrying out the microsatellite instability test.

8. The method of obtaining information according to any one of claims 1 to 7, wherein phosphor integrated dots (PIDs) are used for carrying out the step.
